# EUROPEAN PATENT SPECIFICATION

(11) **EP 4 407 023 B1**
(45) Date of publication and mention of the grant of the patent: **29.10.2025**
(21) Application number: 22946602.4
(22) Date of filing: 30.11.2022
(51) Int. Cl.: C12N 1/20, A61K 35/747, A23L 33/135, A61Q 19/08, C12R 1/225

(54) **LACTOBACILLUS REUTERI FOR PROLONGING LIFESPAN, RESISTING AGING AND REDUCING FAT, AND PRODUCT THEREOF AND USE THEREOF**
LACTOBACILLUS REUTERI ZUR VERLÄNGERUNG DER LEBENSDAUER, BESTÄNDIGKEIT GEGEN ALTERUNG UND REDUZIERUNG VON FETT SOWIE PRODUKT DARAUS UND VERWENDUNG DAVON
LACTOBACILLUS REUTERI POUR PROLONGER LA DURÉE DE VIE, RÉSISTER AU VIEILLISSEMENT ET RÉDUIRE LA GRAISSE, ET PRODUIT ASSOCIÉ ET UTILISATION ASSOCIÉE

(43) Date of publication of application: 31.07.2024
(73) Proprietor: Aiage Life Science Corporation Ltd., Nanning, Guangxi 530200 (CN)
(72) Inventor: LUO, Weifei, Nanning, Guangxi 530200 (CN); MENG, Lili, Nanning, Guangxi 530200 (CN); CHAI, Zhihui, Nanning, Guangxi 530200 (CN); HUANG, Lianfei, Nanning, Guangxi 530200 (CN); LU, Enqiu, Nanning, Guangxi 530200 (CN); XIAO, Guilong, Nanning, Guangxi 530200 (CN); PANG, Shifu, Nanning, Guangxi 530200 (CN); GAN, Caiyu, Nanning, Guangxi 530200 (CN); WEI, Xinli, Nanning, Guangxi 530200 (CN); LIU, Liyuan, Nanning, Guangxi 530200 (CN); LIN, Yanjin, Nanning, Guangxi 530200 (CN); YU, Xiuqi, Nanning, Guangxi 530200 (CN)
(74) Representative: Gassner, Birgitta
(86) International application number: PCT/CN2022/135531
(87) International publication number: WO 2023/240936

(56) References cited:
- CN-A- 110 373 342
- CN-A- 110 452 828
- CN-A- 111 254 090
- CN-A- 114 717 128
- CN-A- 114 717 220
- US-A1- 2015 250 836

## Description

### TECHNICAL FIELD

The present invention belongs to the technical field of screening and application of microorganisms, and specifically relates to *Limosilactobacillus reuteri* for prolonging the lifespan, resisting aging and reducing fat, and product thereof and use thereof.

### BACKGROUND

According to relevant research and survey data, by the end of 2017, there were 240 million elderly people aged 60 and above in China, accounting for 17.3% of the total population. In 2017, the number of new elderly people increased by more than 10 million, and Chinese society is gradually entering the aging stage, which has brought certain pressures and challenges to the national economy and medical system. As the human body ages, many diseases, such as hypertension, etc. may occur in the body. The main cause of hypertension is body obesity. The World Health Organization has declared that obesity is one of the primary health problems. According to the China's "2014 National Physique Monitoring Bulletin", in 2014, the overweight rate of elderly people has exceeded 40%. Moreover, with the growth of the economy and the improvement of living standards, the prevalence of obesity is also increasing, making it gradually become a disease that seriously endangers human health with a continuously increasing incidence rate. At present, the treatment of obesity is still a major challenge, and thus there is an urgent need for a more efficient and safe treatment method for obesity worldwide.

Supplementing probiotics is an obesity treatment idea favored by many researchers at present. Related technologies have shown that probiotics can effectively promote the digestion, absorption and metabolism of human nutrients, improve the body's antioxidant level, and improve the body's immunity. *Limosilactobacillus reuteri* is a lactic acid bacterium that has been reported to exist naturally in the intestines of almost all vertebrates and mammals, which has a certain broad-spectrum antibacterial activity. In 2003, the Ministry of Health of China approved that *Limosilactobacillus reuteri* can be used as a microbial strain for human health care products, and the *Limosilactobacillus reuteri* has been recognized internationally as a novel probiotic lactic acid bacterium, which has high theoretical research and production application value. However, there are few reports on the use of *Limosilactobacillus reuteri* to develop products for prolonging the lifespan, resisting aging and reducing fat or other uses thereof in related fields.

For instance CN 114 717 220 described the Lactobacillus reuteri strain VHProbi E18, deposited at the CCTCC with number M2021153, which is tolerant to artificial gastrointestinal juice, has no toxic effect on the body, has anti-oxidative ability and a skin anti-ageing effect, and functional foods and cosmetics comprising the strain.

### SUMMARY

The present invention aims to solve at least one of the above-mentioned technical problems existing in the prior art. To this end, the present disclosure provides a *Limosilactobacillus reuteri* for prolonging lifespan, resisting aging and reducing fat, and product thereof and use thereof. The present disclosure is based on a strain of *Limosilactobacillus reuteri* A21041 successfully isolated form fecal samples of centenarians in Guangxi, China. It is found that this strain has a better effect of prolonging lifespan than other known *Limosilactobacillus reuteri,* and it also has both gastrointestinal colonization effect and fat reduction effect, and has extremely high development and practical value, providing new directions and ideas for the development and application of products for prolonging lifespan, resisting aging and reducing fat.

In first aspect of the present invention, a strain of *Limosilactobacillus reuteri* is provided. The *Limosilactobacillus reuteri* is *Limosilactobacillus reuteri* A21041, which is taxonomically named *Limosilactobacillus reuteri* and deposited on September 23, 2022 in the Guangdong Microbial Culture Collection Center (GDMCC) (deposit address: 5th Floor, Building 59, No. 100 Xianlie Middle Road, Guangzhou, Guangdong Province, China) with the deposit number of GDMCC No: 62832.

In some embodiments of the present invention, the *Limosilactobacillus reuteri* A21041 is isolated from fecal samples from centenarians in Hepu County, Beihai City, Guangxi, China.

In some embodiments of the present invention, the colony of the *Limosilactobacillus reuteri* A21041 is in the morphology of a round, convex, smooth, neatly-edged and white or milky white colony.

In some embodiments of the present invention, the 16S sequence of the *Limosilactobacillus reuteri* A21041 is as shown in SEQ ID NO: 5.

In some embodiments of the present invention, the whole-gene information of the *Limosilactobacillus reuteri* A21041 is as shown in Table 2.

In some embodiments of the present invention, the screening and isolation method for the *Limosilactobacillus reuteri* A21041 comprises: taking fecal samples of centenarians in Hepu County, Beihai City, Guangxi, China, and placing them into MRS solid medium for culture under anaerobic conditions for 0.2 to 3 days, selecting the colonies in the morphology of round, convex, smooth, neatly-edged and white or milky white in the culture medium, and performing PCR amplification identification, thereby obtaining the *Limosilactobacillus reuteri* A21041.

In some embodiments of the present invention, the culture time is 15 h-24 h.

In some embodiments of the present invention, the culture temperature of the *Limosilactobacillus reuteri* A21041 is 30°C to 37°C, and the rotation speed is 150 rpm to 220 rpm.

In some embodiments of the present invention, *Limosilactobacillus reuteri*-specific amplification primers are used in the PCR amplification identification.

In some embodiments of the present invention, the *Limosilactobacillus reuteri*-specific amplification primers are as shown in SEQ ID NO: 1 and 2.

In some embodiments of the present invention, an amplification system for the PCR amplification identification is:

| Components | Content |
|---|---|
| 10 µM upstream primer F | 1 µL |
| 10 µM downstream primer R | 1 µL |
| PCR amplification solution | 12.5 µL |
| DNA template | 1 µL |
| ddH₂O | Adding to 25 µL |

In some embodiments of the present invention, an amplification procedure for the PCR amplification identification is: 95°C for 3 min; 95°C for 40 s, 56°C for 30 s, 72°C for 1 min, 30 cycles; and 72°C for 8 min.

Of course, those skilled in the art can also reasonably adjust the content and concentration in the PCR amplification system, as well as the amplification procedure, according to actual needs, so as to achieve identification.

In second aspect of the present invention, a product containing the *Limosilactobacillus reuteri* described in the first aspect of the present invention is provided. The product includes food, food additives, feed, feed additives, medicines and cosmetics.

In some embodiments of the present invention, the *Limosilactobacillus reuteri* is *Limosilactobacillus reuteri* A21041.

In some embodiments of the present invention, a mass fraction of the *Limosilactobacillus reuteri* in the product is greater than or equal to 5%.

In some embodiments of the present invention, the mass fraction of the *Limosilactobacillus reuteri* in the product is greater than or equal to 8%.

In some embodiments of the present invention, the mass fraction of the *Limosilactobacillus reuteri* in the product is greater than or equal to 10%.

In some embodiments of the present invention, the mass fraction of the *Limosilactobacillus reuteri* in the product is 5% to 15%.

In some embodiments of the present invention, the product further contains other auxiliary materials.

In some embodiments of the present invention, the auxiliary materials include pharmaceutically acceptable auxiliary agents, food additives, or cosmetic auxiliary materials.

In some embodiments of the present invention, the pharmaceutically acceptable auxiliary agents include, but are not limited to, diluting agents (such as starch, dextrin, sucrose, lactose, mannitol, etc.), absorbing agents (such as calcium sulfate, calcium hydrogen phosphate, etc.), wetting agents (such as ethanol), bonding agents (such as hypromellose, povidone, etc.), disaggregating agents (such as sodium hydroxymethyl starch, crospovidone, etc.), lubricating agents (such as talc, hydrogenated vegetable oil, polyethylene glycol, etc.), coloring agents (such as titanium dioxide, methylene blue, etc.), coating materials, solvents, pH adjusting agents, antibacterial agents (such as sodium sulfite, sodium thiosulfate, etc.), iso-osmotic adjusting agents (such as glucose, sodium chloride, etc.), and chelating agents (such as disodium EDTA).

In some embodiments of the present invention, the food additives include, but are not limited to, coloring agents, enzyme preparations, thickeners, leavening agents, and sweeteners.

In some embodiments of the present invention, the cosmetic auxiliary materials include, but are not limited to, flavors, pigments, preservatives, and antioxidants.

In some embodiments of the present invention, the *Limosilactobacillus reuteri* in the product is in the form including freeze-dried powder, bacterial liquid, and granular inoculant. Of course, it should be understood that those skilled in the art can also reasonably select appropriate forms of *Limosilactobacillus reuteri* for use based on actual needs for use and existing processing techniques, including, but not limited to, the above-mentioned freeze-dried powder, bacterial liquid, and granular inoculant.

In the present invention, the term " freeze-dried powder" refers to a freeze-dried product made by using a vacuum freeze-drying method of a freeze dryer to freeze water in the liquid medicine in advance, and then sublimating the frozen water in the liquid medicine in a vacuum sterile environment.

In the present invention, the term "granular inoculant" refers to a bacteria inoculant used to prevent the powdered bacteria inoculant from directly contacting with the bactericide or chemical fertilizer during use, resulting in reduced effectiveness. It usually refers to a type of inoculant obtained by mixing the bacterial liquid with granular carriers (such as biochar, vermiculite, etc.).

In some embodiments of the present invention, the effective bacterial activity of the *Limosilactobacillus reuteri* in the product is 10⁷ cfu/mL-10⁹ cfu/mL.

Of course, those skilled in the art can reasonably adjust the activity of the *Limosilactobacillus reuteri* in the product according to the actual form thereof, thereby achieving stable technical effects.

In third aspect of the present invention, a preparation method for the product described in the second aspect of the present invention is provided, comprising the following steps: activating and propagating *Limosilactobacillus reuteri,* processing the *Limosilactobacillus reuteri* into a bacterial agent, and mixing the bacterial agent with other auxiliary materials, thereby obtaining the product.

In some embodiments of the present invention, the *Limosilactobacillus reuteri* is *Limosilactobacillus reuteri* A21041.

In some embodiments of the present invention, the activation and propagation conditions of the *Limosilactobacillus reuteri* are as follows: culture time: 15 h-24 h, culture temperature: 30°C-37°C, and the rotation speed: 150 rpm-220 rpm.

In some embodiments of the present invention, the bacterial agent includes, but is not limited to, freeze-dried powder, bacterial solution, and granular inoculant.

In some embodiments of the present invention, the processing technique of the bacterial agent is carried out according to the conventional method in the art. Exemplarily, the preparation method for the freeze-dried powder comprises: freeze-drying the bacterial solution of the *Limosilactobacillus reuteri* A21041, thereby obtaining the freeze-dried powder.

In some embodiments of the present invention, the auxiliary materials include pharmaceutically acceptable auxiliary agents, food additives, or cosmetic auxiliary materials.

In fourth aspect of the present invention, a food additive containing the *Limosilactobacillus reuteri* described in the first aspect of the present invention is provided. The food additive includes *Limosilactobacillus reuteri* A21041 freeze-dried powder, plant extracts, starch hydrolyzed polysaccharide and dietary fiber.

In some embodiments of the present invention, the plant extract includes, but is not limited to, plant essential oil, saponin, alkaloid, polysaccharide, polyphenol and flavonoid compounds.

In some embodiments of the present invention, the plant extract is tea polyphenol and bayberry anthocyanin extract.

In some embodiments of the present invention, the starch hydrolyzed polysaccharide includes, but is not limited to, glycogen, inulin, cellulose, hemicellulose, pectic substances, peptidoglycan, and maltodextrin.

In some embodiments of the present invention, the starch hydrolyzed polysaccharide is maltodextrin.

In some embodiments of the present invention, the dietary fiber includes soluble dietary fiber and insoluble dietary fiber.

In some embodiments of the present invention, the dietary fiber is soluble dietary fiber.

In some embodiments of the present invention, by mass percentage, the food additive includes 5-15% *Limosilactobacillus reuteri* A21041 freeze-dried powder, 15-45% plant extract, 40-60% starch hydrolyzed polysaccharide and 5-15% dietary fiber.

In some embodiments of the present invention, by mass percentage, the food additive includes 5-15% *Limosilactobacillus reuteri* A21041 freeze-dried powder, 5-15% tea polyphenol, 10-30% bayberry anthocyanin extract, 40-60% maltodextrin and 5-15% soluble dietary fiber.

In some embodiments of the present invention, by mass percentage, the food additive includes 8-12% *Limosilactobacillus reuteri* A21041 freeze-dried powder, 10-15% tea polyphenol, 10-15% bayberry anthocyanin extract, 45-55% maltodextrin and 8-12% soluble dietary fiber.

In some embodiments of the present invention, by mass percentage, the food additive includes 10% *Limosilactobacillus reuteri* A21041 freeze-dried powder, 15% tea polyphenol, 15% bayberry anthocyanin extract, 50% maltodextrin and 10% soluble dietary fiber.

In some embodiments of the present invention, the preparation method for the food additive comprises: thoroughly mixing *Limosilactobacillus reuteri* A21041 freeze-dried powder, plant extract, starch hydrolyzed polysaccharide and dietary fiber, thereby obtaining the food additive.

In fifth aspect of the present invention, use of the *Limosilactobacillus reuteri* described in the first aspect of the present invention in the preparation of food, food additives, feed, feed additives, medicines or cosmetics is provided.

In some embodiments of the present invention, the food, food additives, feed, feed additives, medicines or cosmetics have at least one of the following functions (1) to (4):
(1) prolonging lifespan;
(2) promoting colonization of gastrointestinal flora;
(3) resisting aging; and
(4) reducing fat.

In the present invention, a nematode (*Caenorhabditis elegans*) screening platform is used to verify relevant effects. *Caenorhabditis elegans* has a simple structure, a transparent body, is easy to observe, has a short development cycle, is easy to cultivate artificially, and has a highly conserved signaling pathway. As a result, the effect verification accuracy based on *Caenorhabditis elegans* model is high and the experimental cost is low. In the present invention, through the lifespan test, aging resisting test and fat reduction test based on *Caenorhabditis elegans,* it is effectively proved that the *Limosilactobacillus reuteri* A21041 has excellent effects of prolonging lifespan, resisting aging, and reducing body weight and fat simultaneously. Moreover, based on the fact that the *Limosilactobacillus reuteri* A21041 can survive in a simulated gastrointestinal environment, it can be shown that the *Limosilactobacillus reuteri* A21041 has the potential to colonize in the gastrointestinal tract of the body, and fulfills the effects of prolonging lifespan, resisting aging, and reducing body weight and fat stably and permanently.

In sixth aspect of the present invention, a method for prolonging lifespan and resisting aging is provided, comprising the following step: administering an effective dosage of *Limosilactobacillus reuteri* A21041 to a subject in need.

In some embodiments of the present invention, the effective dosage (for humans) is 0.02-0.04 g/kg body weight.

Of course, those skilled in the art can obtain the effective dosage for other animals, including, but not limited to, rats, rabbits, dogs, pigs and monkeys, based on the above-mentioned effective dosage for humans according to a conversion formula.

In some embodiments of the present invention, the administration is oral administration.

In some embodiments of the present invention, the administration frequency is once every 1 to 24 h.

In some embodiments of the present invention, the administration frequency is: once every 1 h, every 2 h, every 4 h, every 6 h, every 8 h, every 12 h or every 24 h.

In seventh aspect of the present invention, a method for reducing fat is provided, comprising the following step: administering an effective dosage of *Limosilactobacillus reuteri* A21041 to a desired subject.

In some embodiments of the present invention, the effective dosage (for humans) is 0.02-0.04 g/kg body weight.

Of course, those skilled in the art can obtain the effective dosage for other animals, including, but not limited to, rats, rabbits, dogs, pigs and monkeys, based on the above-mentioned effective dosage for humans according to conversion formula.

In some embodiments of the present invention, the administration is oral administration.

In some embodiments of the present invention, the administration frequency is once every 1 to 24 h.

In some embodiments of the present invention, the administration frequency is: once every 1 h, every 2 h, every 4 h, every 6 h, every 8 h, every 12 h or every 24 h.

The present invention has the beneficial effects that:
1. The present invention discovers for the first time, a strain of *Limosilactobacillus reuteri* that has an excellent resistance to artificial gastrointestinal fluids and cholate, and also has excellent effects of prolonging the lifespan, anti-aging, and weight loss and fat lowering. Therefore, the related product can be effectively developed on the basis of the strain, which has an extremely high practical value.
2. The product of the present invention contains *Limosilactobacillus reuteri* A21041, which has the potential to effectively colonize in the gastrointestinal tract of the body, and fulfills the effects of prolonging lifespan, anti-aging, and weight loss and fat lowering stably and permanently.

### BRIEF DESCRIPTION OF THE DRAWINGS

FIG. 1 is a specific PCR electrophoresis diagram of *Limosilactobacillus reuteri* A21041 in example 1;
FIG. 2 is a comparative diagram of the effects of *Limosilactobacillus reuteri* A21041 in example 4 and control group OP50 on nematode lifespan;
FIG. 3 is a comparative diagram of the effects of *Limosilactobacillus reuteri* A21041 in example 5 and control group OP50 on nematode aging;
FIG. 4 is a comparative diagram of nematode fat staining between *Limosilactobacillus reuteri* A21041 in example 6 and control group OP50 on day 7;
FIG. 5 is a comparative diagram of nematode fat staining between *Limosilactobacillus reuteri* A21041 in example 7 and control group OP50 on day 5;
FIG. 6 is a diagram showing the effect of *Limosilactobacillus reuteri* A21041 in example 7 on nematode lifespan and fat metabolism gene expression level, and a diagram of related mechanism pathways;
FIG. 7 is a comparative diagram of the effects of commercial *Limosilactobacillus reuteri* 17938 in comparative example 1 and control group OP50 on nematode lifespan;
FIG. 8 is a comparative diagram of nematode fat staining between commercial *Limosilactobacillus reuteri* 17938 in comparative example 2 and control group OP50;
FIG. 9 is a comparativ diagram of the effects of OP50+Metformin (10 mM) in comparative example 3 and control group OP50 on nematode lifespans;
FIG. 10 is a comparative diagram of the colony forms of *Limosilactobacillus reuteri* A21041-B in comparative example 4 and *Limosilactobacillus reuteri* A21041; and
FIG. 11 is a comparative diagram of nematode fat staining between *Limosilactobacillus reuteri* A21041-B in comparative example 4 and control group OP50.

### DETAILED DESCRIPTION

In order to make the purposes, technical solutions and technical effects of the present invention clearer, the present invention will be further described in detail below in conjunction with specific embodiments. It should be understood that the specific embodiments described in this specification are only intended to explain the present invention and are not intended to limit the present invention.

The experimental materials and reagents used, unless otherwise specified, are all conventional consumables and reagents available from commercial sources.

In the following examples, the formula of the MRS solid medium used is as follows: peptone 10 g/L, beef extract powder 5 g/L, yeast extract powder 4 g/L, glucose 20 g/L, dipotassium hydrogen phosphate 2 g/L, triammonium citrate 2 g/L, sodium acetate 5 g/L, magnesium sulfate 0.2 g/L, manganese sulfate 0.05 g/L, agar 15 g/L, and Tween-80 1 g/L. After mixing the above components, 1L of deionized water was added, the pH to 6.2 ± 0.2 was adjusted, and sterilizing was carried out at 121°C for 15 min to obtain the MRS solid medium.

### Example 1 Isolation and purification of Limosilactobacillus reuteri A21041

The isolation and purification steps of *Limosilactobacillus reuteri* A21041 in this example of the present invention are specifically as follows:
Fecal samples from centenarians in Hepu County, Beihai City, Guangxi, China, was taken and an appropriate amount of the fecal samples were placed into MRS solid medium. After coating evenly, they were placed in an anaerobic workstation for culture at 37°C under the conditions of 90% nitrogen and 10% carbon dioxide for 2 days. After the culture was completed, colonies in the morphology of round, convex, smooth, and neatly-edged and white or milky white colonies on the agar plates were selected and placed into a small amount of MRS liquid medium. After mixing evenly, the mixed solution was transferred into a PCR tube, and placed in a 37 ^{°}C incubator for further culture for 24 h.

PCR amplification solution (2 × Taq PCR StarMix with Loading Dye, purchased from GenStar Biosolutions Co., Ltd.) was added to the cultured PCR tube, and *Limosilactobacillus reuteri*-specific amplification primers was added to perform PCR amplification (the amplification system is as shown in Table 1).

The *Limosilactobacillus reuteri*-specific amplification primers are:
Forward primer F: 5'-GCTTCACTCGCTGCAGTTAA-3' (SEQ ID NO: 1); and
Reverse primer R: 5'-CCAACCAAACCTCGGTCAGA-3' (SEQ ID NO: 2).

**Table 1 Limosilactobacillus reuteri PCR amplification system**

| Components | Content |
|---|---|
| 10 µM upstream primer F | 1 µL |
| 10 µM downstream primer F | 1 µL |
| PCR amplification solution | 12.5 µL |
| DNA template | 1 µL |
| ddH₂O | Adding to 25 µL |

The amplification procedure is: 95°C for 3 min; 95°C for 40 s, 56°C for 30 s, 72°C for 1 min, 30 cycles; 72°C for 8 min.

The amplification product was taken for gel electrophoresis.

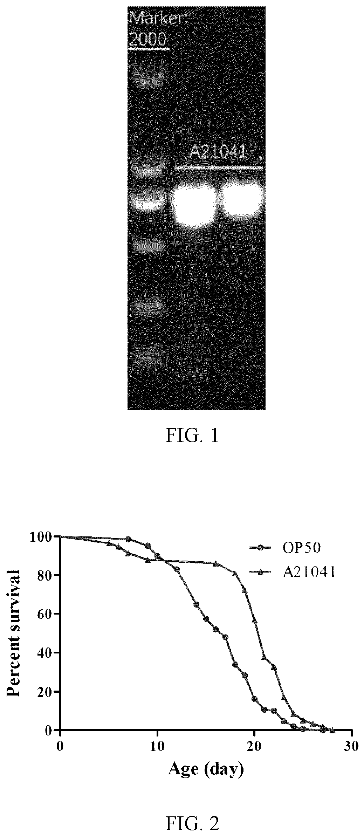

The product was determined to be *Limosilactobacillus reuteri* by polyacrylamide gel electrophoresis, the gel film being as shown in FIG. 1.

Meanwhile, a part of the culture solution was taken and 16S primers were used to perform PCR amplification according to the PCR amplification system shown in Table 1 (the amplification procedure was the same as that in the above example), and the amplification product was sent to Sangon Biotech (Shanghai) Co., Ltd. for sequencing identification.

The nucleotide sequences of the used 16S primers are as follows:
Forward primer 16S-F: 5'-AGAGTTTGATCCTGGCTCAG-3' (SEQ ID NO: 3); and
Reverse primer 16S-R: 5'-TACGGCTACCTTGTTACGACTT-3' (SEQ ID NO: 4).

After sequencing identification, it was confirmed to be *Limosilactobacillus reuteri,* with 16S sequence: 5'-GAGGGACGGGCGGGTGCTATACATGCAAGTCGTACGCACTGGCCCAACTGATTG ATGGTGCTTGCACCTGATTGACGATGGATCACCAGTGAGTGGCGGACGGGTGAG TAACACGTAGGTAACCTGCCCCGGAGCGGGGGATAACATTTGGAAACAGATGCT AATACCGCATAACAACAAAAGCCGCATGGCTTTTGTTTGAAAGATGGCTTTGGC TATCACTCTGGGATGGACCTGCGGTGCATTAGCTAGTTGGTAAGGTAACGGCTTA CCAAGGCGATGATGCATAGCCGAGTTGAGAGACTGATCGGCCACAATGGAACTG AGACACGGTCCATACTCCTACGGGAGGCAGCAGTAGGGAATCTTCCACAATGGG CGCAAGCCTGATGGAGCAACACCGCGTGAGTGAAGAAGGGTTTCGGCTCGTAA AGCTCTGTTGTTGGAGAAGAACGTGCGTGAGAGTAACTGTTCACGCAGTGACG GTATCCAACCAGAAAGTCACGGCTAACTACGTGCCAGCAGCCGCGGTAATACGT AGGTGGCAAGCGTTATCCGGATTTATTGGGCGTAAAGCGAGCGCAGGCGGTTGC TTAGGTCTGATGTGAAAGCCTTCGGCTTAACCGAAGAAGTGCATCGGAAACCGG GCGACTTGAGTGCAGAAGAGGACAGTGGAACTCCATGTGTAGCGGTGGAATGC GTAGATATATGGAAGAACACCAGTGGCGAAGGGCGGCTGTCTGGTCTGCAACTG ACGCTGAGGCTCGAAAGCATGGGTAGCGAACAGGATTAGATACCCTGGTAGTCC ATGCCGTAAACGATGAGTGCTAGGTGTTGGAGGGTTTCCGCCCTTCAGTGCCGG AGCTAACGCATTAAGCACTCCGCCTGGGGAGTACGACCGCAAGGTTGAAACTC AAAGGAATTGACGGGGGCCCGCACAAGCGGTGGAGCATGTGGTTTAATTCGAA GCTACGCGAAGAACCTTACCAGGTCTTGACATCTTGCGCTAACCTTAGAGATAA GGCGTTCCCTTCGGGGACGCAATGACAGGTGGTGCATGGTCGTCGTCAGCTCGT GTCGTGAGATGTTGGGTTAAGTCCCGCAACGAGCGCAACCCTTGTTACTAGTTG CCAGCATTGAGTTGGGCACTCTAGTGAGACTGCCGGTGACAAACCGGAGGAAG GTGGGGACGACGTCAGATCATCATGCCCCTTATGACCTGGGCTACACACGTGCT ACAATGGACGGTACAACGAGTCGCAAACTCGCGAGAGTAAGCTAATCTCTTAAA GCCGTTCTCAGTTCGGACTGTAGGCTGCAACTCGCCTACACGAAGTCGGAATCG CTAGTAATCGCGGATCAGCATGCCGCGGTGAATACGTTCCCGGGCCTTGTACAC ACCGCCCGTCACACCATGGGAGTTTGTAACGCCCAAAGTCGGTGGCCTAACCTT TATGGAGGGGACCGCGCCCCCCCCTATAACGC-3' (SEQ ID NO: 5). The confirmed culture solution was purified by plate streaking twice, and the purified single colonies were selected for repeated culture to obtain bacterial liquid. The bacterial liquid was mixed with 30% glycerol preservation solution at a volume ratio of 1: 1 in a strain cryopreservation tube, and the strain cryopreservation tube was stored in a -80°C refrigerator.

Further, the *Limosilactobacillus reuteri* obtained in the above example was subjected to whole-gene sequencing (performed by Annuouda Gene Technology (Beijing) Co., Ltd.). The results are as shown in Table 2.

**Table 2 Whole-gene sequencing results of Limosilactobacillus reuteri A21041**

| Sample ID | Contigs | Total lenght /bp | GC ratio (%) | CDS/ piece | Gene/ piece |
|---|---|---|---|---|---|
| A21041 | 128 | 2011591 | 38.77 | 1977 | 2048 |

The *Limosilactobacillus reuteri* obtained in the above example (named *Limosilactobacillus reuteri* A21041), which is taxonomically named *Limosilactobacillus reuteri* and deposited on September 23, 2022 in Guangdong Microbial Culture Collection Center (GDMCC) (deposit address: 5th Floor, Building 59, No. 100 Xianlie Middle Road, Guangzhou, Guangdong, China) with the deposit number of GDMCC No: 62832.

### Example 2 Test of resistance to artificial gastrointestinal fluids and cholates of Limosilactobacillus reuteri A21041

The specific experimental steps are as follows:
The above-mentioned *Limosilactobacillus reuteri* A21041 was taken for overnight culture and activation, and the bacterial liquid was diluted to a concentration of OD600=1. The bacterial liquid was inoculated into an artificial gastric fluid with pH = 2.5 (obtained by diluting 1 mol/mL of dilute hydrochloric acid with water) according to an inoculation amount of 10% by volume. 100 µL of solutions were taken after 0 h, 1 h and 3 h respectively and coated onto the MRS agar plates, which were cultured in a biochemical incubator at 37°C for 48 h, and the number of single colonies was counted.

By means of the same method as above, the bacterial liquid was inoculated into an artificial intestinal fluid with pH = 8 (pH 6.8, 6.8 g of potassium dihydrogen phosphate was taken, adding with 500 mL of water to dissolve, and 0.1 mol/L of sodium hydroxide solution was used to adjust the pH to 6.8. Trypsin aqueous solution (dissolving 10 g of trypsin in water) was added. It diluted to 1000 mL with water) according to an inoculation amount of 10% by volume. 100 µL of solutions were taken after 2 h, 4 h, 6 h and 8 h respectively and coated onto the MRS agar plates, which were cultured in a biochemical incubator at 37°C for 48 h, and the number of single colonies was counted.

Meanwhile, the bacterial liquid was inoculated into MRS liquid medium of 0.3 g/L and 0.5 g/L of ox bile salt according to an inoculation amount of 10% by volume respectively. 100 µL of solutions were taken after 0 h, 2 h and 24 h respectively and coated onto the MRS agar plates, which were cultured in a biochemical incubator at 37°C for 48 h, and the number of single colonies was counted.

The survival rate of *Limosilactobacillus reuteri* A21041 in artificial gastrointestinal fluids and ox cholate solutions based on the number of single colonies in the above experiment was calculated.

The results showed that the survival rate of *Limosilactobacillus reuteri* A21041 was 90% after being treated with artificial gastric fluid with pH = 2.5 for 3 h, and the survival rate was about 80% after being treated with artificial intestinal fluid with pH = 8.0 for 8 h. The survival rates of *Limosilactobacillus reuteri* A21041 after being treated with 0.3 g/L and 0.5 g/L of ox cholate solutions for 24 h were 260% and 80% respectively. Therefore, *Limosilactobacillus reuteri* A21041 in the example of the present invention has the potential to colonize in the human gastrointestinal tract for a long time.

### Example 3 Safety evaluation of Limosilactobacillus reuteri A21041

In order to ensure the safety of *Limosilactobacillus reuteri* A21041 in various uses, safety evaluation was performed on the strain in this example, and the detection institution was Guangdong Detection Center of Microbiology. According to the determination criteria in Appendix A of the Guideline on Safety Inspection and Evaluation of Strains Used in Health Food Raw Materials (2020) in China, *Limosilactobacillus reuteri* A21041 has no adverse effects on the general health of mice. There was no statistically significant difference in weight and other indicators compared with the control group. Therefore, under the conditions of the example of the present invention, the sample is not pathogenic. Meanwhile, whole-genome sequencing was performed on A21041, and whole-genome analysis was performed through Diamond (version 2.1.0.4) and RGI (version 5.2.0) software. According to the virulence factor database standards (matching degree ≥ 85% and e-value < 10⁻⁵ means non-toxic genes or pathogenic factors), the matching degree of A21041 is ≥ 95%, and the e-value is < 10⁻⁵. Therefore, from the genetic level evaluation, A21041 is non-toxic. Therefore, *Limosilactobacillus reuteri* A21041 is a safe probiotic.

### Example 4 Lifespan test of Limosilactobacillus reuteri A21041

### (1) Culture of Limosilactobacillus reuteri A21041, Escherichia coli OP50 and Caenorhabditis elegans:

*Limosilactobacillus reuteri* A21041 in MRS liquid medium and *Escherichia coli* OP50 in LB liquid medium were cultured under the culture conditions of: temperature: 37°C, time: 20 h, rotation speed: 200 rpm. Bacterial liquids of *Limosilactobacillus reuteri* A21041 and Escherichia coli OP50 (both bacterial liquid concentrations are 10⁷ CFU/mL) were taken and spread onto nematode growth medium (NGM) for overnight culture.

The formula of the NGM culture medium is as follows: 3 g of sodium chloride, 17 g of agar powder, 2.5 g of peptone, and 975 mL of deionized water. After mixing the above components thoroughly, seal with tin foil. After high-pressure steam sterilization for 20 min, cool the water bath to 55°C. Aseptically add the following sterile solutions: 1 mL of 1M CaCl₂, 1 mL of 1M MgSO₄, 25 mL of 1M KPO₄ buffer, 1 mL of 5 mg/mL cholesterol (dissolved in 95% ethanol).

The thawed nematodes (Caenorhabditis elegans) was centrifuged and added to the NGM with OP50 respectively, and placed in a 20°C incubator for culture for 3 days.

### (2) Nematode synchronization treatment:

M9 buffer was used to suspend the cultured nematodes in step (1). They were sucked into the culture tubes. Lysis solution (5N NaOH by mass and 5% sodium hypochlorite solution by volume) was added to each tube for 6 min lysis, centrifuge for 1 min at 3500 r/min, discard the supernatant was discarded, and washed 4 times repeatedly with M9 buffer. Centrifuge again and the supernatant was discarded. The precipitate was transferred to the NGM for overnight culture at a constant temperature of 20°C to obtain L1 stage nematode larvae. After washing with M9 buffer and centrifuging once, the nematode larvae was transferred to the NGM agar plate containing OP50, and cultured at 20°C for 28-30 h to obtain synchronized nematodes, namely L4 stage nematodes.

### (3) Lifespan test:

The obtained synchronized nematodes were randomly divided into two groups (control group (OP50) and experimental group (A21041)), with 150 nematodes in each group. The day when the L4 stage nematodes were picked was recorded as day 0. During the experiment, for the nematodes, the NGM containing OP50 and A21041 was changed every 2 days and lifespans were counted until all nematodes died. Abnormally dead nematodes were excluded in the statistics. Statistical analysis was performed using GraphPad Prism 5, and the significant differences between multiple groups were compared using the one-way ANOVA; and the differences between every two groups using the t-test method were analyzed. P < 0.05 means that there is a significant difference, and P < 0.01 means that there is a very significant difference.

The results are as shown in FIG. 1. It can be found that compared with the OP50 control group, the lifespans of nematodes were significantly prolonged after feeding A21041 (the average lifespans of the OP50 control group and A21041 experimental group were 18 days (d) and 21.5 days (d) respectively). Compared with the OP50 control group, A21041 prolonged the lifespans of nematodes by 19.66% (P < 0.05).

### Example 5 Aging resisting test

*Limosilactobacillus reuteri* A21041, *Escherichia coli* OP50 and *Caenorhabditis elegans* were cultured according to the method in the above example, and nematodes were synchronized.

The synchronized nematodes were randomly divided into two groups (control group (OP50) and experimental group (A21041)), with 150 nematodes in each group. The day when L4 stage nematodes were picked was recorded as day 0. During the experiment, for the nematodes, the NGM containing OP50 and A21041 was changed every 2 days. The frequencies of head swing of the nematodes were counted within 30 s one by one on the NGM agar plate containing M9 buffer when the nematodes were raised to day 5, day 10 and day 15. The swing of the nematode's head from one side to the other side and back to the original position was counted as one swing.

The results are as shown in FIG. 2. It can be found that on day 10 and day 15 (middle-aged and elderly stages of nematodes), *Limosilactobacillus reuteri* A21041 can significantly increase the frequency of head swing of nematodes and increase the vitality of nematodes. Therefore, *Limosilactobacillus reuteri* A21041 can significantly delay nematode aging.

### Example 6 Fat reduction test

*Limosilactobacillus reuteri* A21041, *Escherichia coli* OP50 and *Caenorhabditis elegans* were cultured according to the method in the above example, and nematodes were synchronized.

100 synchronized nematodes were transferred to agar plates coated with OP50 and A21041 for culture. Culture for 7 days, and the plates were changed every other day. The nematodes were re-suspended in 4% paraformaldehyde after washing three times with chilled M9 buffer on day 7. Shake slightly at room temperature for 1 hour, centrifuge at 3000-4000 rpm for 1 min, the supernatant was removed and washed twice with M9 buffer. The nematodes were re-suspended in a mixed PBS solution containing 60% isopropyl alcohol and 0.01% Triton X-100 by volume, and incubated for 15 min; after the nematodes settle, the isopropyl alcohol was removed, 1 mL of 40% Oil Red O stain was added, and incubated on a 25°C shaker for 1 to 2 h. Remove the dye, and wash twice with M9 buffer. 200 µL of M9 buffer was added, and pictures of the nematodes were taken one by one under an inverted fluorescence microscope. The image data was counted using Image J, and the fat particle degradation rate data was counted using GraphPad Prism 5. P < 0.05 means that there is a significant difference, and P < 0.01 means that there is a very significant difference.

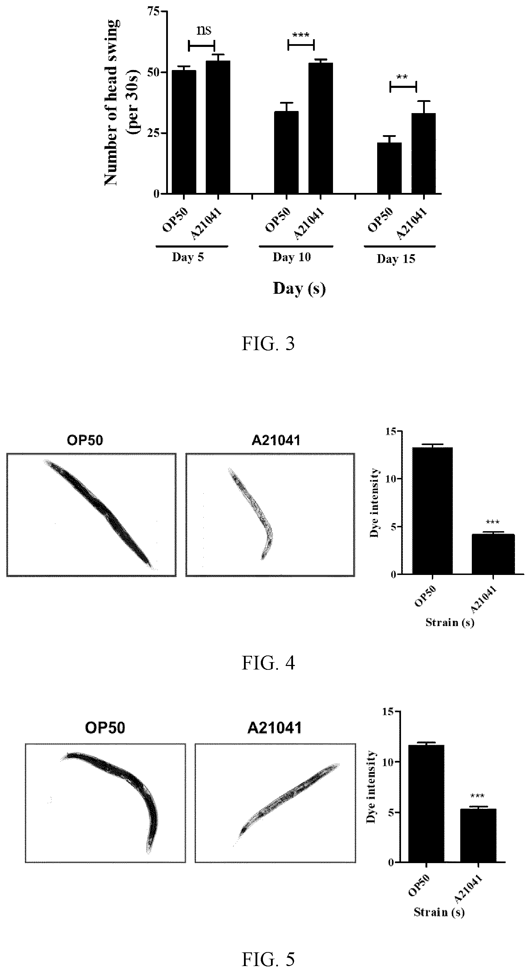

The results are as shown in FIG. 3. It can be found that compared with the OP50 control group, the fat particles of the nematodes in the experimental group fed with *Limosilactobacillus reuteri* A21041 were obviously lighter in color and the fat particles were smaller, indicating that *Limosilactobacillus reuteri* A21041 had a significant inhibitory effect on nematode fat particles, with an inhibition rate up to 68.77% (P < 0.01).

The above experiment was repeated and the culture time was adjusted to 5 days.

The results are as shown in FIG. 4. On day 5 after feeding A21041, after staining the nematodes, it was found that compared with the OP50 control group, the fat particles of the nematodes in the experimental group fed with *Limosilactobacillus reuteri* A21041 were obviously lighter in color and the fat particles were smaller, indicating that feeding *Limosilactobacillus reuteri* A21041 for only 5 days can have a significant inhibitory effect on nematode fat particles, with an inhibition rate of 54.47%.

### Example 7 Fluorescence quantitative PCR determination of effects of Limosilactobacillus reuteri A21041 on lifespan and fat metabolism gene loci

*Limosilactobacillus reuteri* A21041, *Escherichia coli* OP50 and *Caenorhabditis elegans* were cultured according to the method in the above example, and nematodes were synchronized.

800 synchronized nematodes were transferred to agar plates coated with OP50 and A21041 for culture. Culture for 5 days, and the plates were changed every other day. The nematodes were washed 1-2 times with M9 buffer on day 5. The nematodes after freeze-drying were ground with liquid nitrogen, and total RNA was extracted from the nematodes according to the instructions of the total RNA extraction kit (purchased from Promega (Beijing) Biotech Co., Ltd.). Then, equal amounts of RNA samples were taken from each group for reverse transcription PCR amplification according to the instructions of the reverse transcription kit (purchased from Tiangen Biotech (Beijing) Co., Ltd.). The PCR amplification products was stored at -20°C for later use. The primer pairs of each gene shown in Table 3 were used to perform conventional fluorescence quantitative PCR, and 2-ΔΔCt was used for data statistics.

**Table 3 Fluorescent quantitative PCR detection locus and corresponding primer sequence information**

| Locus | | Nucleotide sequences |
|---|---|---|
| *act* | act-1F | 5'-CTACGAACTTCCTGACGGACAAG-3' (SEQ ID NO:6) |
| | act-1R | 5'-CCGGCGGACTCCATACC-3' (SEQ ID NO:7) |
| *daf-16* | daf-16F | 5'-CTAACTTCAAGCCAATGCCACTA-3' (SEQ ID NO:8) |
| | daf-16R | 5'-TCCAGCTTGACTCAGCTCATGTC-3' (SEQ ID NO:9) |
| *sod* | sod-3F | 5'-CTCCAAGCACACTCTCCCAG-3' (SEQ ID NO:10) |
| | sod-3R | 5'-TCCCTTTCGAAACAGCCTCG-3' (SEQ ID NO:11) |
| *hsf* | hsf-1F | 5'-TTTGCATTTTCTCGTCTCTGTC-3' (SEQ ID NO:12) |
| | hsf-1R | 5'-TCTATTTCCAGCACACCTCGT-3' (SEQ ID NO:13) |
| *hsp-* | hsp-16.2F | 5'-GGTGCAGTTGCTTCGAATCTT-3' (SEQ ID NO:14) |
| *16.2* | hsp-16.2R | 5'-TCTTCCTTGAACCGCTTCTTTC-3' (SEQ ID NO:15) |
| *nhr-80* | nhr-80 F | 5'-AATTCCGATTTCCAGCTTCTTC-3' (SEQ ID NO:16) |
| | nhr-80 R | 5'-TCTGCAGATTTGGTGCATACTATAA-3' (SEQ ID NO:17) |
| *fat-6* | fat-6 F | 5'-GGCAAACCGTGATTTTCACATT-3' (SEQ ID NO:18) |
| | fat-6 R | 5'-TCACGAGCCCATTCGATGAC-3' (SEQ ID NO:19) |
| *daf-12* | daf-12 F | 5'-TCCAATGCCAGCTGAAACAACACC-3' (SEQ ID NO:20) |
| | daf-12 R | 5'-TGGAATGGCTGACACGGTTGAATG-3' (SEQ ID NO:21) |
| *fard-1* | fard-1 F | 5'-CGCATTCGCCAAGAGAAACC-3' (SEQ ID NO:22) |
| | fard-1 R | 5'-ACGTTGACATTGTCTCGGATGA-3' (SEQ ID NO:23) |
| *skn-1* | skn-1F | 5'-TACAGAACGTCCAACCACATC-3' (SEQ ID NO:24) |
| | skn-1R | 5'-GCCCTTCTCTCCAGCAATATC-3' (SEQ ID NO:25) |
| *daf-2* | daf-2F | 5'-GAGCTTCGGAGTTGTTCTCTATG-3'(SEQ ID NO:26) |
| | daf-2R | 5'-CTTCCGGGCCATTCCAATATAA-3' (SEQ ID NO:27) |
| *age-1* | age-1F | 5'-GTGACGAGCTTCGATCGATTAG-3' (SEQ ID NO:28) |
| | age-1R | 5'-GGACGATGGCTTTGTCGATTA-3' (SEQ ID NO:29) |

The results are as shown in FIG. 5. It can be seen that the expression of nhr-80 and daf-12 genes was significantly up-regulated, especially nhr-80, which was up-regulated nearly 2 times compared with the control group. Therefore, the above results can show that prolonging nematode lifespan by A21041 may be related to the signaling pathways regulated by nhr-80 and daf-12 genes, that is, related to the nuclear hormone receptor signaling pathway.

### Example 8 Preparation of product for prolonging lifespan, anti-aging and reducing fat based on Limosilactobacillus reuteri A21041

The *Limosilactobacillus reuteri* A21041 strain was taken for fermentation and propagation and then centrifuged and freeze-dried the bacterial liquid after fermentation and propagation to obtain *Limosilactobacillus reuteri* A21041 freeze-dried powder. Compound according to the following formula to obtain a food additive or dietary supplement containing *Limosilactobacillus reuteri* A21041.

Based on the dosage for humans and calculated by weight percentage, the formula of the food additive or dietary supplement containing *Limosilactobacillus reuteri* A21041 is as follows: 10% *Limosilactobacillus reuteri* A21041 freeze-dried powder, 15% tea polyphenol (purchased from Thankcome Biological Science and Technology Co., Ltd.), 15% bayberry anthocyanin extract (purchased from Thankcome Biological Science and Technology Co., Ltd.), 50% maltodextrin and 10% soluble dietary fiber (purchased from Thankcome Biological Science and Technology Co., Ltd.). The preparation method includes: thoroughly mixing the above raw materials to obtain the product.

### Comparative example 1

In this comparative example, the commercial *Limosilactobacillus reuteri* strain 17938 was isolated from Senbao probiotic drops, and the culture method and nematode lifespan test method were the same as those in the above example.

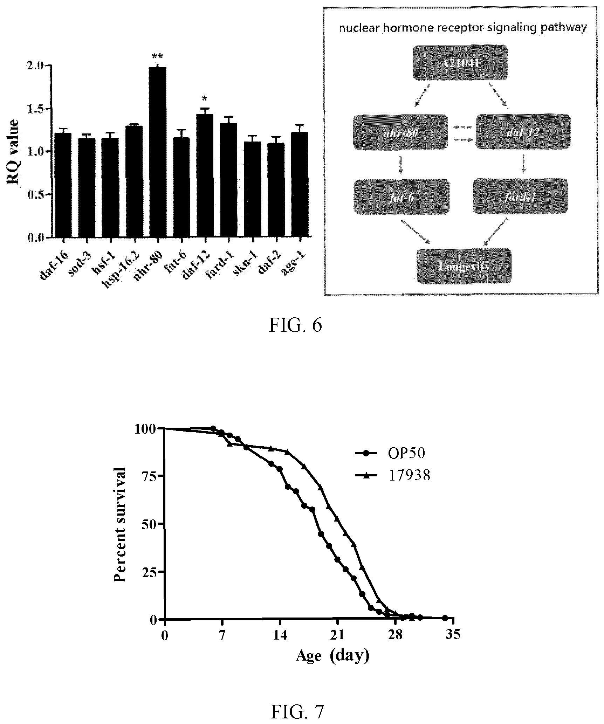

The effect comparison results are as shown in FIG. 6. It can be found that compared with the OP50 control group, the lifespans of nematodes can be prolonged after feeding *Limosilactobacillus reuteri* 17938 (the average lifespans of the OP50 control group and the *Limosilactobacillus reuteri* 17938 experimental group were 18 d and 20 d respectively). Compared with the OP50 control group, 17938 prolonged the lifespans of nematodes by 11.11%. However, compared with *Limosilactobacillus reuteri* A21041, the lifespan prolonging effect of *Limosilactobacillus reuteri* A21041 is more significant, indicating that *Limosilactobacillus reuteri* A21041 has a stronger lifespan prolonging effect than the existing *Limosilactobacillus reuteri.*

### Comparative example 2

In this comparative example, the source and culture method of the commercial *Limosilactobacillus reuteri* 17938 strain were the same as those in the above-mentioned comparative example 1, and the nematode fat particle test method is the same as that in the above-mentioned example.

The effect comparison results are as shown in FIG. 7. It can be found that the color of the fat particles of nematodes fed with the commercial *Limosilactobacillus reuteri* 17938 strain is not significantly different from that of the control group. The inhibition rate is only 33.85%, and there is no fat reduction effect. *Limosilactobacillus reuteri* A21041 has a significant fat reduction effect.

### Comparative example 3

Because metformin, a drug for treating diabetes mellitus type 2, has the effect of prolonging the lifespans of nematodes, it was used as a positive drug in this comparative example.

In this comparative example, *Limosilactobacillus reuteri* A21041, *Escherichia coli* OP50 and *Caenorhabditis elegans* were cultured according to the method in the above example, and nematodes were synchronized.

Synchronized nematodes were randomly divided into two groups (control group (OP50) and positive group (OP50+Metformin (final concentration: 10mM)), with 150 nematodes in each group. The day when L4 stage nematodes were picked was recorded as day 0. During the experiment, for the nematodes, the NGM containing OP50 was changed every 2 days and their lifespans were counted until all nematodes died. Abnormally dead nematodes were excluded in the statistics. Statistical analysis was performed using GraphPad Prism 5, and the significant differences between multiple groups were compared using the one-way ANOVA; and and the differences between every two groups were analyzed using the t-test method. P < 0.05 means that there is a significant difference, and P < 0.01 means that there is a very significant difference.

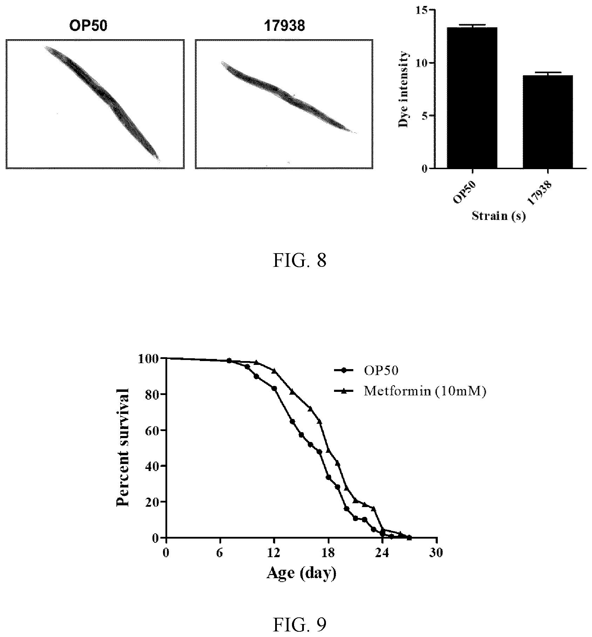

The effect comparison results are as shown in FIG. 8. It can be found that compared with the control group, the lifespans of nematodes can be prolonged after feeding OP50+Metformin (10 mM) (the average lifespans of the OP50 control group and the OP50+Metformin (10 mM) positive group were 18 d and 19 d respectively). Compared with the control group, Metformin (10 mM) prolonged the lifespans of nematodes by 5.56%. However, compared with the feeding effect of *Limosilactobacillus reuteri* A21041, the prolonging effect of lifespans of nematodes fed with *Limosilactobacillus reuteri* A21041 was significantly better than that of the positive group added with Metformin.

### Comparative example 4

From the samples in example 1, a strain of *Limosilactobacillus reuteri* A21041-B was also isolated. Observed under a microscope, the *Limosilactobacillus reuteri* A21041-B was different from the *Limosilactobacillus reuteri* A21041 in colony morphology, the edge of the colony of A21041-B was thinner and the overall colony was more transparent; and the edge of the colony of A21041 was thicker, as shown in FIG. 9. In addition, the 16S sequenced nucleotide sequence of A21041-B is the same as SEQ ID NO: 5, indicating that it is also a strain of *Limosilactobacillus reuteri.*

According to the operations in the above example, A21041-B was subjected to the same isolation and purification, artificial gastrointestinal fluid resistance test, artificial bile salt resistance test and fat particle test.

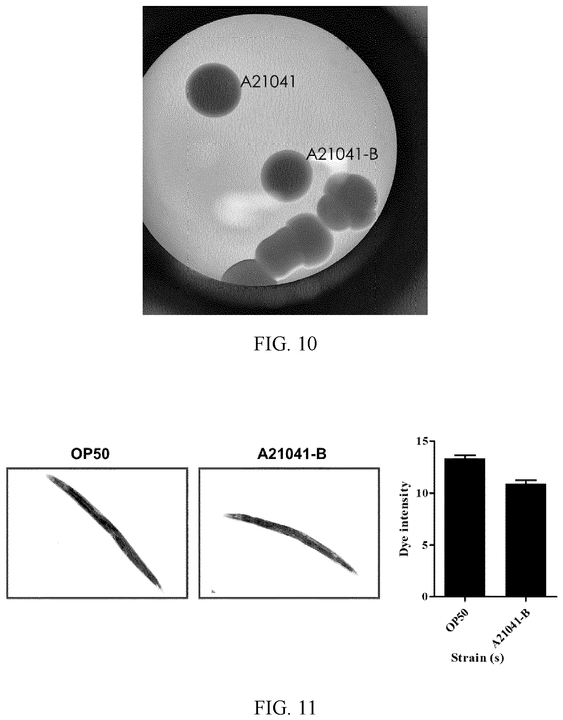

The results are as shown in FIG. 10. It can be found that although A21041-B also has a certain effect of resisting artificial gastrointestinal fluid and bile salt, its inhibitory effect on nematode fat particles is not as good as *Limosilactobacillus reuteri* A21041, and the inhibition rate is only 18.24%.

In summary, the above results show that among the existing *Limosilactobacillus reuteri,* only *Limosilactobacillus reuteri* A21041 has excellent effects of prolonging lifespan, anti-aging, and reducing body weight and fat simultaneously. Moreover, based on the fact that *Limosilactobacillus reuteri* A21041 can survive in a simulated gastrointestinal environment, it can be shown that the *Limosilactobacillus reuteri* A2104 has the potential to colonize in the gastrointestinal tract of the body, and fulfills the effects of prolonging lifespan, resisting aging, and reducing body weight and fat stably and permanently.

The above examples are preferred examples of the present invention, but the embodiments of the present invention are not limited to the above examples. Any other changes, modifications, substitutions, combinations, etc. made without departing from the spirit and principles of the present invention should be equivalent substitutions, and are all included in the protection scope of the present invention.

## Claims

1. A strain of *Limosilactobacillus reuteri,* wherein the *Limosilactobacillus reuteri* is *Limosilactobacillus reuteri* A21041, which is taxonomically named *Limosilactobacillus reuteri* and deposited on September 23, 2022 in the Guangdong Microbial Culture Collection Center with the deposit number of GDMCC No: 62832.

2. A product containing the *Limosilactobacillus reuteri* according to claim 1, wherein the product comprises food, food additives, feed, feed additives, medicines and cosmetics.

3. The product according to claim 2, wherein a mass fraction of the *Limosilactobacillus reuteri* in the product is greater than or equal to 5%.

4. The product according to claim 2, wherein the mass fraction of the *Limosilactobacillus reuteri* in the product is greater than or equal to 8%.

5. The product according to claim 2, wherein the mass fraction of the *Limosilactobacillus reuteri* in the product is greater than or equal to 10%.

6. The product according to claim 2, wherein the product further contains other auxiliary materials, and the auxiliary materials comprise pharmaceutically acceptable auxiliary agents, food additives, and cosmetic auxiliary materials.

7. The product according to claim 2, wherein the *Limosilactobacillus reuteri* in the product is in a form including freeze-dried powder, bacterial liquid, and a granular inoculant.

8. The product according to claim 2, wherein the food, food additives, feed, feed additives, medicines and cosmetics have at least one of the following functions (1) to (4):
(1) prolonging lifespan;
(2) promoting colonization of gastrointestinal flora;
(3) resisting aging; and
(4) reducing fat.

9. A preparation method for the product according to any one of claims 2 to 7, comprising the following steps: activating and propagating *Limosilactobacillus reuteri,* processing the *Limosilactobacillus reuteri* into a bacterial agent, and mixing the bacterial agent with other auxiliary materials, thereby obtaining the product.

10. A food additive containing the *Limosilactobacillus reuteri* according to claim 1, wherein the food additive comprises *Limosilactobacillus reuteri* A21041 freeze-dried powder, a plant extract, starch hydrolyzed polysaccharide and dietary fiber.

11. The food additive according to claim 10, wherein by mass percentage, the food additive comprises 5-15% *Limosilactobacillus reuteri* A21041 freeze-dried powder, 15-45% plant extract, 40-60% starch hydrolyzed polysaccharide and 5-15% dietary fiber.

12. Use of the *Limosilactobacillus reuteri* according to claim 1 in preparation of food, food additives, feed, feed additives, medicines and cosmetics.

13. The use according to claim 12, wherein the food, food additives, feed, feed additives, medicines and cosmetics have at least one of the following functions (1) to (4):
(1) prolonging lifespan;
(2) promoting colonization of gastrointestinal flora;
(3) resisting aging; and
(4) reducing fat.

## Patentansprüche

1. *Limosilactobacillus reuteri*-Stamm, wobei der *Limosilactobacillus reuteri Limosilactobacillus reuteri* A2l04l ist, welcher taxonomisch *Limosilactobacillus reuteri* bezeichent wird und am 23. September 23 2022 in dem Guangdong Microbial Culture Collection Center mit der folgenden GDMCC-Hinterlegungsnummer Nr. hinterlegt wurde: 62832.

2. Produkt, das den *Limosilactobacillus reuteri* nach Anspruch l enthält, wobei das Produkt Lebensmittel, Lebensmittelzusatzstoffe, Futtermittel, Futtermittelzusatzstoffe, Medikamente und Kosmetika umfasst.

3. Produkt nach Anspruch 2, wobei ein Masseanteil des *Limosilactobacillus reuteri* in dem Produkt größer als oder gleich 5 % ist.

4. Produkt nach Anspruch 2, wobei der Masseanteil des *Limosilactobacillus reuteri* in dem Produkt größer als oder gleich 8% ist.

5. Produkt nach Anspruch 2, wobei der Masseanteil des *Limosilactobacillus reuteri* in dem Produkt größer als oder gleich 10% ist.

6. Produkt nach Anspruch 2, wobei das Produkt ferner andere Hilfsmaterialien enthält und die Hilfsmaterialien pharmazeutisch annehmbare Hilfsmittel, Lebensmittelzusatzstoffe Kosmetikhilfsmittel umfassen.

7. Produkt nach Anspruch 2, wobei das *Limosilactobacillus reuteri* in dem Produkt in einer Form vorliegt, die gefriergetrocknetes Pulver, Bakterienflüssigkeit und ein körniges Impfmittel beinhaltet.

8. Produkt nach Anspruch 2, wobei die Lebensmittel, Lebensmittelzusatzstoffe, Futtermittel, Futtermittelzusatzstoffe, Medikamente und Kosmetika mindestens eine der folgenden Funktionen (1) bis (4) aufweisen:
(1) die Lebenserwartung verlängern;
(2) die Besiedlung der gastrointestinalen Flora fördern;
(3) dem Altern widerstehen und
(4) Fett reduzieren.

9. Herstellungsverfahren für das Produkt nach einem der Ansprüche 2 bis 7, umfassend die folgenden Schritte: Aktivieren und Verbreiten von *Limosilactobacillus reuteri,* Verarbeiten des *Limosilactobacillus reuteri* zu einem bakteriellen Mittel und Vermischen des bakteriellen Mittels mit anderen Hilfsmaterialien, wodurch das Produkt erhalten wird.

10. Lebensmittelzusatzstoff, das das *Limosilactobacillus reuteri* nach Anspruch l enthält, wobei der Lebensmittelzusatzstoff gefriergetrocknetes *Limosilactobacillus reuteri* A21041-Pulver, ein Pflanzenextrakt, aus Stärke hydrolisiertes Polysacharid und Ballaststoff umfasst.

11. Lebensmittelzusatzstoff nach Anspruch 10, wobei, in Massenanteilen in Prozent, der Lebensmittelzusatzstoff 5-15 % gefriergetrocknetes *Limosilactobacillus reuteri* A2l041-Pulver, 15-45 % Pflanzenextrakt, 40-60 % aus Stärke hydrolisiertes Polysacharid und 5-15 % Ballaststoff umfasst.

12. Verwendung von *Limosilactobacillus reuteri* nach Anspruch l bei der Herstellung von Lebensmitteln, Lebensmittelzusatzstoffen, Futtermitteln, Futtermittelzusatzstoffen, Medikamenten und Kosmetika.

13. Verwendung nach Anspruch 12, wobei die Lebensmittel, Lebensmittelzusatzstoffe, Futtermittel, Futtermittelzusatzstoffe, Medikamente und Kosmetika mindestens eine der folgenden Funktionen (1) bis (4) aufweisen:
(1) die Lebenserwartung verlängern;
(2) die Besiedlung der gastrointestinalen Flora fördern;
(3) dem Altern widerstehen und
(4) Fett reduzieren.

## Revendications

1. Souche de *Limosilactobacillus reuteri,* dans laquelle le *Limosilactobacillus reuteri* est le *Limosilactobacillus reuteri* A21041, qui est taxonomiquement appelé *Limosilactobacillus reuteri* et a été déposé le 23 septembre 2022 dans le Guangdong Microbial Culture Collection Centre avec le numéro de dépôt de GDMCC n° : 62832.

2. Produit contenant le *Limosilactobacillus reuteri* selon la revendication 1, dans lequel le produit comprend des aliments, des additifs alimentaires, des aliments pour animaux, des additifs alimentaires pour animaux, des médicaments et des produits cosmétiques.

3. Produit selon la revendication 2, dans lequel une fraction massique du *Limosilactobacillus reuteri* dans le produit est supérieure ou égale à 5 %.

4. Produit selon la revendication 2, dans lequel la fraction massique du *Limosilactobacillus reuteri* dans le produit est supérieure ou égale à 8 %.

5. Produit selon la revendication 2, dans lequel la fraction massique du *Limosilactobacillus reuteri* dans le produit est supérieure ou égale à 10 %.

6. Produit selon la revendication 2, dans lequel le produit contient d'autres matériaux auxiliaires, et les matériaux auxiliaires comprennent des agents auxiliaires pharmaceutiquement acceptables, des additifs alimentaires et des matériaux auxiliaires cosmétiques.

7. Produit selon la revendication 2, dans lequel le *Limosilactobacillus reuteri* dans le produit est sous une forme incluant une poudre lyophilisée, un liquide bactérien et un inoculant granulaire.

8. Produit selon la revendication 2, dans lequel les aliments, les additifs alimentaires, les aliments pour animaux, les additifs alimentaires pour animaux, les médicaments et les produits cosmétiques ont au moins l'une des fonctions (1) à (4) suivantes :
(1) la prolongation de la durée de vie ;
(2) la favorisation de la colonisation de la flore gastrointestinale ;
(3) la résistance au vieillissement ; et
(4) la réduction des graisses.

9. Méthode de préparation du produit selon l'une quelconque des revendications 2 à 7, comprenant les étapes suivantes : l'activation et la propagation du *Limosilactobacillus reuteri,* le traitement du *Limosilactobacillus reuteri* dans un agent bactérien, et le mélange de l'agent bactérien avec d'autres matériaux auxiliaires, ce qui permet l'obtention du produit.

10. Additif alimentaire contenant le *Limosilactobacillus reuteri* selon la revendication 1, dans lequel l'additif alimentaire comprend une poudre lyophilisée de *Limosilactobacillus reuteri* A21041, un extrait végétal, un polysaccharide hydrolysé d'amidon et une fibre alimentaire.

11. Additif alimentaire selon la revendication 10, dans lequel en pourcentage massique, l'additif alimentaire comprend 5 à 15 % de poudre lyophilisée de *Limosilactobacillus reuteri* A21041, 15 à 45 % d'extrait végétal, 40 à 60 % de polysaccharide hydrolysé d'amidon et 5 à 15 % de fibre alimentaire.

12. Utilisation du *Limosilactobacillus reuteri* selon la revendication 1 dans la préparation d'aliments, d'additifs alimentaires, d'aliments pour animaux, d'additifs alimentaires pour animaux, de médicaments et de produits cosmétiques.

13. Utilisation selon la revendication 12, dans laquelle les aliments, les additifs alimentaires, les aliments pour animaux, les additifs alimentaires pour animaux, les médicaments et les produits cosmétiques ont au moins l'une des fonctions (1) à (4) suivantes :
(1) la prolongation de la durée de vie ;
(2) la favorisation de la colonisation de la flore gastrointestinale ;
(3) la résistance au vieillissement ; et
(4) la réduction des graisses.
